# EUROPEAN PATENT APPLICATION

(11) **EP 3 005 888 A1**
(43) Date of publication of application: **13.04.2016**
(21) Application number: 13885615.8
(22) Date of filing: 31.05.2013
(51) Int. Cl.: A24F 47/00

(54) **ELECTRONIC CIGAR**

(71) Applicant: KIMREE HI-TECH INC., Road Town, Tortola (VG)
(72) Inventor: LIU, Qiuming, Shenzhen Guangdong 518000 (CN)
(74) Representative: Bosch, Matthias
(86) International application number: PCT/CN2013/076608
(87) International publication number: WO 2014/190560

(57) **Abstract**

An electronic cigar. The electronic cigar comprises a smoking part (1) and a main body part (2) connected to the smoking part (1). The electronic cigar also is provided with an outer cover layer (3). The smoking part (1) and the main body part (2) form coaxially a junction (4). The outer cover layer (3) covers the junction (4) and bordering areas adjacent to the junction (4). The electronic cigar that employs said structure has an aesthetic appearance and allows the smoking part (1) and the mam body part (2) to be firmly connected.

## Description

### TECHNICAL FIELD OF THE INVENTION

The present invention relates to electronic heating products, and more particularly to an electronic cigar.

### RELATED ART

At present, an electronic cigar usually has a connecting seam between the smoking part and the main body part. Such connecting seam has not only made the appearance unbeautiful, but also made the electronic cigar easily opened under the action of an external force since the main body part and the smoking part are detachably connected.

### SUMMARY

Aiming at the drawbacks in the prior art that the electronic cigar is unbeautiful and easily opened under the action of external force, an object of the present invention is to provide an electronic cigar which provides beautiful appearance and secure connection between the smoking part and the main body part.

The above and other objects are achieved by the invention as described below.

An electronic cigar is provided, which comprises a smoking part and a main body part connected with the smoking part; a junction is formed coaxially between the smoking part and the main body part; the electronic cigar has an outer cover layer covering the junction and bordering areas adjacent to the junction.

An uneven pattern and/or a character are provided on an outer surface of the outer cover layer.

Two ends of the outer cover layer are docked to cover the junction and the bordering areas adjacent to the junction.

A concave area is defined on the smoking part and the main body part for placing the outer cover layer.

A shape of the concave area is adapted to a shape of the outer cover layer, a depth of the concave area is the same as a thickness of the outer cover layer, and the outer cover layer is embedded in the concave area.

The outer surface of the outer cover layer is coated with a non-slip layer.

The outer surface of the outer cover layer is covered with a transparent protective layer, and an outer surface of the transparent protective layer is coated with a non-slip layer.

The transparent protective layer is embedded in the concave area, and a total thickness of the outer cover layer and the transparent protective layer is equal to a depth of the concave area.

A length of the outer cover layer covering the smoking part and a length of the outer cover layer covering the main body part are respectively 2mm ~ 15mm.

The smoking part and the main body part are detachably connected.

The smoking part and the main body part are connected together by means of threaded connection, buckle connection, magnetic connection, or interference fit.

The outer cover layer is made of tipping paper, copperplate paper, plastic paper or synthetic paper.

The smoking part is only a single suction nozzle, and the main body part includes an atomizing assembly and a power supply for powering the atomizing assembly.

The smoking part includes a suction nozzle and an atomizing assembly, and the main body part includes a power supply for powering the atomizing assembly.

By implementing the electronic cigar according to the present invention, the following beneficial effects can be achieved. By arranging an outer cover layer for the electronic cigar and covering the junction and the bordering areas adjacent to the junction with the outer cover layer, the electronic cigar looks beautiful. Moreover, the outer cover layer helps to strengthen the connection between the smoking part and the main body part.

### BRIEF DESCRIPTION OF THE DRAWINGS

The present invention will be described in company with the drawings, wherein:
FIG. 1 is an overall structural diagram of an electronic cigar according to one embodiment of the present invention;
FIG. 2 is an overall structural diagram of an electronic cigar according to another embodiment of the present invention.
FIG. 3 is an exploded diagram of an electronic cigar and an outer cover layer according to the present invention.
FIG. 4 is a structural diagram of an electronic cigar with a protective layer according to the present invention.

### DETAILED DESCRIPTION

The embodiments of the present invention will now be described in detail in company with the drawings to make the technical features, objective and effects of the present invention be understood more completely.

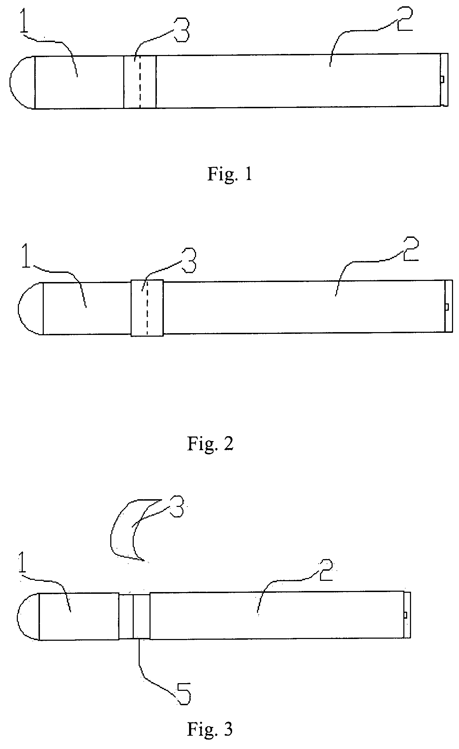

As shown in FIGS. 1 and 2, an electronic cigar according to the present invention comprises a smoking part 1 and a main body part 2 connected with the smoking part 1. A junction 4 is formed coaxially between the smoking part 1 and the main body part 2. The electronic cigar has an outer cover layer 3 covering the junction 4 and bordering areas adjacent to the junction.

Understandably, the smoking part 1 may either only include a suction nozzle, or may include the suction nozzle and an atomizing assembly. If the smoking part only includes the suction nozzle, the main body part 2 includes the atomizing assembly and a power supply powering the atomizing assembly. If the smoking part 1 includes both the suction nozzle and the atomizing assembly, the main body part 2 only includes the power supply for powering the atomizing assembly, in this case, the axial length of the smoking part 1 is longer and the atomizing assembly is located inside the smoking part 1.

The outer surface of the outer cover layer 3 may be printed with an uneven pattern and/or a character. Of course, the outer surface of the outer cover layer 3 may also be printed with a flat pattern and/or a character. The outer cover layer 3 may be made of tipping paper, copperplate paper, plastic paper or synthetic paper. In a preferred embodiment of the present invention, a plastic paper is used as the outer cover layer 3. Specifically, the outer cover layer 3 may be stickers, logo, etc.

Due to the utilization of the outer cover layer 3, the connection between the smoking part1 and the main body part 2 is strengthened.

The two ends of the outer cover layer 3 are docked to cover the junction 4 and the bordering areas adjacent to the junction. Since the two ends of the outer cover layer 3 are docked, the outer cover layer 3 can easily be detached and changed. The outer cover layer 3 covers in way of pasting or buckling. Optionally, a double-sided adhesive or glue is provided between the outer cover layer 3 and the smoking part 1 and the main body part 2.

In order to ensure that it is hard for the electronic cigar to fall off from the hands in a humid environment or water environment, the outside surface of the outer cover layer 3 is further coated with a non-slip layer.

The outer cover layer 3 can directly cover the outer surface of the electronic cigar (as shown in FIG. 2, the outer cover layer 3 protrudes from the outer surface of the electronic cigar).

As shown in FIG. 3, a concave area 5 is defined on the smoking part and the main body part for placing the outer cover layer 3. A shape of the concave area 5 is adapted to a shape of the outer cover layer 3, a depth of the concave area 5 is the same as a thickness of the outer cover layer 3, and the outer cover layer 3 is embedded in the concave area. Thus the outer surface of the electronic cigar is neat and the appearance looks beautiful.

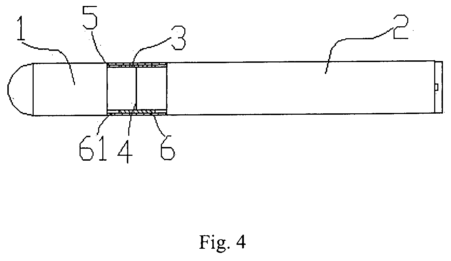

As shown in FIG. 4, the outer surface of the outer cover layer 3 may be covered with a transparent protective layer 6, and in this case, the outer surface of the transparent protective layer 6 is coated with a non-slip layer.
Understandably, when the transparent protective layer 6 is provided, the total thickness of the outer cover layer 3 and the transparent protective layer 6 is not limited. The total thickness may be equal to the depth of the concave area 5, and the protective layer 6 is embedded in the concave area 5, as shown in Fig. 4. Thus, the outer surface of the protective layer 6 is flush with the whole outer surface of the electronic cigar and the appearance looks beautiful. Also, the transparent protective layer 6 may protrude from the outer surface of the electronic cigar, as shown in FIG. 2.

In the above embodiments, the transparent protective layer 6 may be a plastic cement layer, a silicone rubber layer or a plastic layer. When the transparent protective layer 6 is elastic, it can be made as a sleeve and is fitted in the concave area 5 which is a ring groove. Alternatively, the transparent protective layer 6 may be directly pasted on the concave area 5 and the outer cover layer 3. Preferably, the axial length of the transparent protective layer 6 is greater than or equal to the axial length of the outer cover layer 3.

The two ends of the transparent protective layer 6 can also be docked, so that the transparent protective layer 6 is easily disassembled, and further the outer cover layer 3 is easily disassembled.

The transparent protective layer 6 can also be clamped outside the smoking part 1 and the main body part 2.

Specifically, two axial protrusion parts 61 protruding from two sides of the outer cover layer 3 are respectively defined on two sides of the transparent protective layer 6. A convex portion is defined on an inner wall of the two protrusion parts 61, a concave portion is defined on the smoking part 1 and the main body part 2 corresponding to the convex portion, and the convex portion is engaged with the concave portion. Alternatively, a concave portion is defined on the inner wall of the two protrusion parts 61, a convex portion is defined on the smoking part 1 and the main body part 2, and the convex portion is engaged with the concave portion (not shown in the drawings).

By arranging the transparent protective layer 6 outside the outer cover layers 3, it can prevent the outer cover layer 3 from being scratched or torn. By setting the non-slip layer outside the outer cover layer 3, it can prevent the electronic cigar from easily sliding from hands in the humid environment or other water environment.

In the above embodiments, two lengths that the outer cover layer 3 is respectively sleeved on the smoking part 1 and the main body part 2 are both ranged from 2mm to 15mm. The lengths of the two protrusion parts 61 located at two ends of the transparent protective layer 6 are ranged from 2mm to 10mm. According to a preferred embodiment of the present invention, the two lengths that the outer cover layer 3 is respectively sleeved on the smoking part 1 and the main body part 2 are both 10mm. The two lengths of the two protrusion parts 61 located at the two ends of the transparent protective layer 6 are 5 mm. In this case, it ensures both the beauty of the appearance and firm connection between the smoking part 1 and the main body part 2.

The smoking part 1 and the main body part 2 may either be integrally formed or detachably connected. When the smoking part 1 and the main body part 2 are detachably connected, they are connected by means of threaded connection, interference fit, buckle connection, magnetic connection, wedge action or other suitable means.

When connected by means of the threaded connection, the smoking part 1 is provided with an internal thread, and the main body part 2 is provided with an external thread. Alternatively, the smoking part 1 is provided with the external thread, and the main body part 2 is provided with the internal thread.

When connected by means of interference fit, the smoking part 1 is inserted into the main body part 2 in way of interference fit. Alternatively, the main body part 2 is inserted into the smoking part 1 in way of interference fit.

In summary, the electronic cigar is provided with the outer cover layer 3 which covers the junction 4 of the smoking part1 and the main body part 2and the bordering areas adjacent to the junction 4. Thus, the electronic cigar looks beautiful, and the connection between the smoking part and the main body part is strengthened.

While the invention has been described based on the illustrated embodiments, the invention is not limited to them. The above embodiments are illustrative, but not limitative. Under the teaching of the present invention, one skilled in the art may obtain other embodiments without departing from the spirit of the present invention and the scope of the claims. All these embodiments fall into the protection scope of the present invention.

## Claims

1. An electronic cigar comprising:
a smoking part;
a main body part connected with the smoking part;
a junction formed coaxially between the smoking part and the main body part; and
an outer cover layer covering the junction and bordering areas adjacent to the junction.

2. The electronic cigar according to claim 1, wherein an uneven pattern and/or a character are provided on an outer surface of the outer cover layer.

3. The electronic cigar according to claim 1, wherein two ends of the outer cover layer are docked to cover the junction and the bordering areas adjacent to the junction

4. The electronic cigar according to claim 3, wherein a concave area is defined on the smoking part and the main body part for placing the outer cover layer.

5. The electronic cigar according to claim 4, wherein a shape of the concave area is adapted to a shape of the outer cover layer, a depth of the concave area is the same as a thickness of the outer cover layer, and the outer cover layer is embedded in the concave area.

6. The electronic cigar according to claim 1 or 5, wherein an outer surface of the outer cover layer is coated with a non-slip layer.

7. The electronic cigar according to claim 4, wherein an outer surface of the outer cover layer is covered with a transparent protective layer, and an outer surface of the transparent protective layer is coated with a non-slip layer.

8. The electronic cigar according to claim 7, wherein the protective layer is embedded in the concave area, and the total thickness of the outer cover layer and the protective layer is equal to a depth of the concave area.

9. The electronic cigar according to claim 1, wherein a length of the outer cover layer covering the smoking part and a length of the outer cover layer covering the main body part are respectively 2mm ~ 15mm.

10. The electronic cigar according to claim 1, wherein the smoking part and the main body part are detachably connected.

11. The electronic cigar according to claim 10, wherein the smoking part and the main body part are connected together by means of threaded connection, buckle connection, magnetic connection, or interference fit.

12. The electronic cigar according to claim 1, wherein the outer cover layer is made of tipping paper, copperplate paper, plastic paper or synthetic paper.

13. The electronic cigar according to claim 1, wherein the smoking part is only a single suction nozzle, and the main body part includes an atomizing assembly and a power supply for powering the atomizing assembly.

14. The electronic cigar according to claim 1, wherein the smoking part includes a suction nozzle and an atomizing assembly, and the main body part includes a power supply for powering the atomizing assembly.
